Europäisches Patentamt

European Patent Office

Office européen des brevets

(11) Veröffentlichungsnummer: **0 210 645**

·A2

## (12) EUROPÄISCHE PATENTANMELDUNG

(21) Anmeldenummer: 86110443.8

(22) Anmeldetag: 29.07.86

(51) Int. Cl.⁴ **C07K 15/00** , C07K 3/00 , C12N 15/00 , G01N 33/68 , A61K 37/00

(30) Priorität: 01.08.85 DE 3527568

(43) Veröffentlichungstag der Anmeldung:
04.02.87 Patentblatt 87/06

(84) Benannte Vertragsstaaten:
AT BE CH DE FR GB IT LI LU NL SE

(71) Anmelder: Bissendorf Peptide GmbH
Burgwedeler Strasse 25
D-3002 Wedemark 2(DE)

(72) Erfinder: Hesch, Rolf-Dieter, Prof. Dr.
Gartenweg 12
D-3004 Isernhagen 2(DE)

(74) Vertreter: Werner, Hans-Karsten, Dr. et al
Deichmannhaus am Hauptbahnhof
D-5000 Köln 1(DE)

(54) Synthetische Peptidrezeptoren, Verfahren zur Herstellung und Verwendung derselben.

(57) Synthetische Peptidrezeptoren bestehen aus einer oder enthalten eine Aminosäuresequenz, die komplementär oder analog komplementär ist zur Sequenz des jeweiligen Peptides. Sie können löslich, an Träger gebunden oder an Marker gebunden sein. Sie werden nach der Festphasenmethode oder biotechnologisch hergestellt. Sie können präparativ, diagnostisch und therapeutisch verwendet werden.

EP 0 210 645 A2

## Synthetische Peptidrezeptoren, Verfahren zur Herstellung und Verwendung derselben

Gegenstand der vorliegenden Erfindung sind synthetische Peptidrezeptoren, Verfahren zur Herstellung und Verwendung derselben, insbesondere zu diagnostischen, präparativen und therapeutischen Zwecken.

Hormone und Mediatoren, die aus mehr oder weniger langen Peptiden bestehen, wirken im allgemeinen erst, wenn sie vom Rezeptor aufgenommen sind. Diese Rezeptoren sind im allgemeinen hochspezifisch. Bereits geringfügige Veränderungen in der Aminosäuresequenz des Hormons oder Mediators sind in der Lage, die Spezifität negativ zu beeinflussen. Bisher war die Struktur der Rezeptoren weitgehend unbekannt, zumal sie meist unlöslich sind und in die Zellwand oder -Membranen eingebaut sind.

Bost et al. haben die Theorie aufgestellt, daß zwischen Peptiden und ihren Rezeptoren eine Beziehung besteht, wonach komplementäre DNS, sofern sie in der Richtung von 5' nach 3' transscribiert werden, Peptide oder Proteine kodieren, die mit den regulär, d.h. nicht am komplementären Strang positiv abgelesenen Peptiden in Wechselwirkung treten. Diese Theorie wurde getestet und an zwei synthetisierten Peptiden, nämlich dem komplementären ACTH und dem komplementären gamma-Endorphin, bestätigt (vgl. Proc. Natl. Acad. Sci. USA 82, 1985, Seite 1372 bis 1375). Bei der Diskussion dieser Ergebnisse wurde zwar festgestellt, daß diese Erkenntnis eine Reihe von interessanten wissenschaftlichen Folgerungen nach sich ziehen könne und weitere Erkenntisse über Peptid-Peptid-Wechselwirkungen untersucht werden könnten, jedoch wurde nicht erkannt, daß sich hieraus auch eine Reihe außerordentlich bedeutungsvoller praktischer Konsequenzen ziehen lassen.

So ist es beispielsweise möglich, derartige komplementäre oder analog komplementäre Peptide dadurch in größeren Mengen herzustellen, daß die komplementären oder analog komplementären Aminosäuresequenzen aus dem komplementären RNS-Strang des jeweiligen Peptides berechnet und nach der Festphasenmethode synthetisiert werden. Weiterhin ist es möglich, den komplementären RNS-Strang des jeweiligen Peptides gentechnologisch in einen Vektor einzubauen und biotechnologisch zu exprimieren. Auf diese Weise erhält man in größeren Mengen und hoher Reinheit synthetische Peptidrezeptoren, welche aus einer Aminosäuresequenz bestehen bzw. diese enthalten, die komplementär ist zur Sequenz des jeweiligen Peptides. Analog komplementär bedeutet, daß die komplementäre Sequenz durch Einbau ähnlich hydrophober oder hydrophiler Aminosäuren an typischer Stelle des Moleküls mit Methoden des Peptiddesigns auch so modifiziert werden kann, daß ihre amphiphilische Sekundärstruktur erhalten, stabilisiert oder fixiert wird. Insofern sind auch derartige modifizierte analog komplementäre Peptide synthetische Peptidrezeptoren.

Diese synthetischen Peptidrezeptoren sind allgemein zunächst einmal löslich, so daß sie wesentlich leichter gehandhabt und weiterverwendet werden können. Es ist aber auch möglich, sie an einen festen Träger zu binden und in dieser Form für diagnostische und präparative Zwecke einzusetzen. Als Träger kommen alle üblichen festen Träger wie Polystyrol, Glas, Dextrane, aber auch biologische Membranen und Lipid-Vesikel in Frage.

Weiterhin ist es möglich, an diese synthetischen Peptidrezeptoren übliche Marker zu binden und sie in dieser Form diagnostisch einzusetzen. Weiterhin ist es möglich, diese synthetischen Peptidrezeptoren zur Diagnostik und therapeutischen Behandlung von Zuständen der Überproduktion von Hormonen oder Mediatoren einzusetzen sowie gegebenenfalls auch bei akuten allergischen Reaktionen, da sie blockierend in das Geschehen einwirken. Schließlich können die synthetischen Peptidrezeptoren zur Erzeugung von polyclonalen und/oder monoclonalen Antikörpern verwendet werden, die dann spezifisch gegen natürliche Rezeptoren wirken. Derartige Antikörper können dann wiederum für diagnostische und therapeutische Zwecke eingesetzt werden und damit völlig neue Wege und Möglichkeiten eröffnen.

Von besonderer Bedeutung sind solche synthetischen Peptidrezeptoren, die eine komplementäre oder analog komplementäre Aminosäuresequenz zu relativ kurzkettigen Peptiden aufweisen. Diese kurzkettigen Peptidrezeptoren lassen sich in besonders einfacher Weise nach der Festphasenmethode synthetisieren, sofern die Aminosäuresequenz des Peptides bzw. des RNS-Stranges bekannt ist. Hieraus läßt sich nämlich der komplementäre RNS-Strang bzw. die komplementäre oder analog komplementäre Aminosäuresequenz berechnen. Bei längerkettigen Peptiden, d.h. bei Kettenlängen über 40 bis 50 Aminosäureresten, bereitet die Festphasenmethode bereits so erhebliche Schwierigkeiten, daß es eigentlich nur noch möglich ist, den komplementären RNS-Strang des jeweiligen Peptides zu synthetisieren, gentechnologisch in einen Vektor einzubauen und biotechnologisch zu exprimieren. Derartige längerkettige Peptide sind im allgemeinen auch nicht auf ihrer gesamten Kettenlänge spezifisch gegenüber dem natürlichen Rezeptor.

Die erfindungsgemäßen synthetischen Peptidrezeptoren für längerkettige Peptide enthalten somit auch für die Spezifität nicht relevante Abschnitte. Trotzdem wird es im allgemeinen einfacher sein, die volle komplementäre Aminosäuresequenz zu synthetisieren, anstatt die für die spezifische Peptid-Peptid-Bindung relevanten Teile ausfindig zu machen.

Für die spezifische Peptid-Peptid-Bindung zwischen synthetischen Peptidrezeptoren und den jeweiligen positiv abgelesenen Peptiden ist es im einfachsten Falle ausreichend, wenn der synthetische Peptidrezeptor exakt aus der Aminosäuresequenz besteht, die komplementär ist zur RNS des jeweiligen Peptides, d.h. an der RNS negativ abgelesen wurde. Insbesondere wenn jedoch die synthetischen Peptidrezeptoren an einen Träger oder einen Marker gebunden werden sollen, kann es durchaus zweckmäßig sein, die Aminosäuresequenz zu verlängern, um dadurch eine negative Beeinflussung der Spezifität der Peptid-Peptid-Bindung zu vermeiden. In diesen Fällen enthält der synthetische Peptidrezeptor eine Aminosäuresequenz, die komplementär ist zur Sequenz des jeweiligen Peptides.

Sofern die synthetischen Peptidrezeptoren an einen Träger gebunden werden, können sie sowohl diagnostisch als auch präparativ zur Bindung des jeweiligen natürlichen Peptides verwendet werden, beispielsweise mittels der sogenannten Affinitätschromatographie. Diagnostisch kann ein natürliches Peptid durch einen an einen Träger gebundenen synthetischen Peptidrezeptor in üblicher Weise erfaßt werden, z.B. mittels Antikörpern oder markierten Antikörpern. Als Markierung kommt beispielsweise radioaktive Markierung, ein Enzym oder eine fluoreszierende Gruppe in Frage.

Bei der therapeutischen Verwendung der synthetischen Peptidrezeptoren können diese in entsprechend hoch gereinigter Form injiziert werden, so daß sie dann überhöhte Mengen von Hormonen oder Mediatoren kompetitv gegenüber den natürlichen Rezeptoren inhibieren. Vorzugsweise wird man für diesen Zweck lösliche Peptidrezeptoren verwenden, die ausschließlich aus der Aminosäuresequenz bestehen, die komplementär oder analog komplementär ist zur Sequenz des jeweiligen Hormons oder Mediators. Derartige Lösungen können auch zur Diagnostik und Differentialdiagnostik herangezogen werden, da hierdurch überschüssige Mengen von Hormonen oder Mediatoren auch analytisch maskiert werden.

Von außerordentlicher Bedeutung ist die Verwendung der synthetischen Peptidrezeptoren zur Erzeugung von polyclonalen und/oder monoclonalen Antikörpern, die spezifisch gegen natürliche Rezeptoren wirken. Derartige Antikörper können zunächst wiederum diagnostisch eingesetzt werden zur Darstellung und Bestimmung der Rezeptoren auf Zellen oder zellbiologischem Material. Weiterhin können sie therapeutisch eingesetzt werden zum Blokkieren von Rezeptoren, beispeilsweise von spezifischen Rezeptoren auf Tumorzellen. Besonders geeignet sind diese Antikörper für die sogenannte Flow-Cytometrie, die für die Zelldiagnostik zunehmend an Bedeutung gewinnt. Mit Hilfe derartiger Antikörper wird es möglich sein, eine exakte Quantifizierung der Expression von Oberflächenrezeptoren festzustellen, die eine wesentliche Zelleigenschaft sind. Therapeutisch können diese Antikörper eingesetzt werden gegen die Überproduktion von Hormonen, zumindest in der akuten Phase, die oft gefährlich ist, z.B. bei akutem Hyperparathyreodismus und Insulinom.

Beispiele für interessante synthetische Peptidrezeptoren sind das komplementäre Peptid zum hPTH, d.h. dem humanen Parathormon, oder zum CRF, d.h. dem "corticotropin releasing factor", also dem hypothalamischen Faktor, welcher die Freisetzung von ACTH aus der Hypophyse stimuliert. Weitere interessante synthetische Peptidrezeptoren sind das komplementäre Peptid zum hANaP - (humanes atriales natriuretisches Peptid) sowie zum GHRH (growth hormone releasing hormone) d.h. dem hypothalamischen Faktor, welcher die Freisetzung von Wachstumshormon aus der Hypophyse stimuliert. Weitere interessante synthetische Peptidrezeptoren sind die komplementären Peptide zu allen bekannten Transformations-Wachstumsfaktoren, insbesondere Interleukine, Interferone, Plateletderived Growth Factor, Epidermal Growth Factor, Transferrin, Erythropoitin, Tumor-nekrosis-faktor, Colony-stimulating-faktor, Faktor VIII, sowie alle Onkogen-Proteine. Ferner sind von besonderem Interesse alle Neuropeptide, sofern sie nicht schon weiter oben erwähnt wurden.

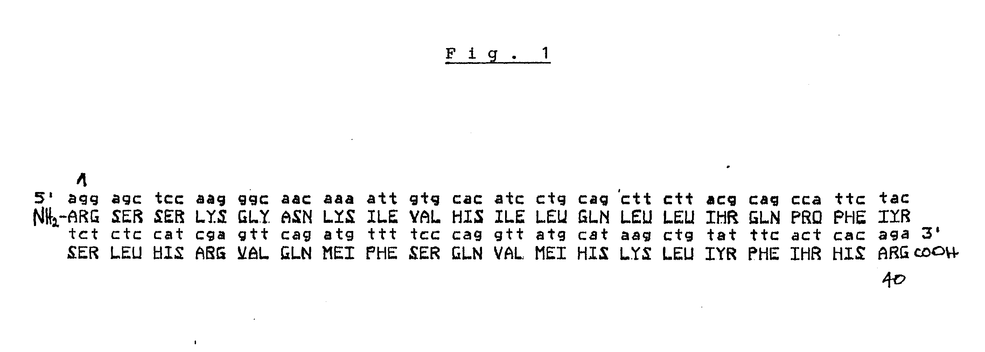

Als typisches Beispiel für einen bevorzugten erfindungsgemäßen synthetischen Peptidrezeptor wird in der anliegenden Fig. 1 die errechnete Aminosäuresequenz für das komplementäre Peptid zum (1-38) hPTH dargestellt. Dieses Peptid wird nach der Festphasenmethode (Merrifield) synthetisiert und wirkt als synthetischer Rezeptor.

Das synthetische Peptid erwies sich als stark hydrophob und lipophil. Es zeigte daher die typischen Eigenschaften eines Membran-Rezeptorproteins. Das an der Oberfläche eines festen Trägers immobilisierte synthetische Peptid war in der Lage, mit 131-Jod markiertes Parathormon aus einer Lösung zu adsorbieren. Das gebundene, markierte Parathormon konnte danach einfach durch unmarkiertes Parathormon verdrängt werden.

## Ansprüche

1. Synthetische Peptidrezeptoren enthaltend eine Aminosäuresequenz, die komplementär oder analog komplementär ist zur RNS des jeweiligen Peptides.

2. Synthetische Peptidrezeptoren gemäß Anspruch 1, dadurch gekennzeichnet, daß sie aus der Aminosäuresequenz bestehen, die komplementär ist zur Sequenz des jeweiligen Peptides.

3. Synthetische Peptidrezeptoren gemäß Anspruch 1 oder 2, dadurch gekennzeichnet, daß sie löslich sind.

4. Synthetische Peptidrezeptoren gemäß Anspruch 1 oder 2, dadurch gekennzeichnet, daß sie an einen festen Träger gebunden sind.

5. Synthetische Peptidrezeptoren gemäß Anspruch 1 oder 2, dadurch gekennzeichnet, daß sie an einen Marker gebunden sind.

6. Verfahren zur Herstellung von synthetischen Peptidrezeptoren bestehend aus einer oder enthaltend eine Aminosäuresequenz, die komplementär oder analog komplementär ist zur Sequenz des jeweiligen Peptides, dadurch gekennzeichnet, daß die komplementäre oder analog komplementäre Aminosäuresequenz aus dem komplementären RNS-Strang des jeweiligen Peptides berechnet und nach der Festphasenmethode synthetisiert wird.

7. Verfahren zur Herstellung von synthetischen Peptidrezeptoren bestehend aus einer oder enthaltend eine Aminosäuresequenz, die komplementär oder analog komplementär ist zur Sequenz des jeweiligen Peptides, dadurch gekennzeichnet, daß der komplementäre RNS-Strang des jeweiligen Peptides gentechnologisch in einen Vektor eingebaut und biotechnologisch exprimiert wird.

8. Verwendung von synthetischen Peptidrezeptoren bestehend aus einer oder enthaltend eine Aminosäuresequenz, die komplementär oder analog komplementär ist zur Sequenz des jeweiligen Peptides zum qualitativen und/oder quantitativen Nachweis des jeweiligen Peptides.

9. Verwendung gemäß Anspruch 8, dadurch gekennzeichnet, daß die synthetischen Peptidrezeptoren an einen festen Träger gebunden sind.

10. Verwendung von synthetischen Peptidrezeptoren bestehend aus einer oder enthaltend eine Aminosäuresequenz, die komplementär oder analog komplementär ist zur Sequenz des jeweiligen Peptides in trägergebundener Form zur analytischen Charakterisierung oder präparativen Gewinnung des jeweiligen Peptides.

11. Verwendung von synthetischen Peptidrezeptoren bestehend aus einer oder enthaltend eine Aminosäuresequenz, die komplementär oder analog komplementär ist zur Sequenz des jeweiligen Peptides zur Diagnostik und therapeutischen Behandlung von Zuständen der Überproduktion von Hormonen oder Mediatoren.

12. Verwendung von synthetischen Peptidrezeptoren bestehend aus einer oder enthaltend eine Aminosäuresequenz, die komplementär oder analog komplementär ist zur Sequenz des jeweiligen Peptides zur Erzeugung von polyclonalen und/oder monoclonalen Antikörpern gegen natürliche Rezeptoren.

0 210 645

Fig. 1

5' agg agc tcc aag ggc aac aaa att gtg cac atc ctg cag ctt ctt acg cag cca ttc tac
NH₂-ARG SER SER LYS GLY ASN LYS ILE VAL HIS ILE LEU GLN LEU LEU THR GLN PRO PHE TYR
tct ctc cat cga gtt cag atg ttt tcc cag gtt atg cat aag ctg tat ttc act cac aga 3'
SER LEU HIS ARG VAL GLN MET PHE SER GLN VAL MET HIS LYS LEU TYR PHE THR HIS ARG COOH

40